# EUROPÄISCHE PATENTSCHRIFT

(11) **EP 0 441 184 B1**
(45) Veröffentlichungstag und Bekanntmachung des Hinweises auf die Patenterteilung: **19.10.1994**
(21) Anmeldenummer: 91100867.0
(22) Anmeldetag: 24.01.1991
(51) Int. Cl.: A61M 27/00, A61M 1/00

(54) **Absaugvorrichtung für medizinische Zwecke**
Suction device for medical purposes
Dispositif de succion à usage médical

(30) Priorität: 27.01.1990 DE 4002373
(43) Veröffentlichungstag der Anmeldung: 14.08.1991
(73) Patentinhaber: Laabs, Walter, Dr. med., D-26419 Schortens (DE); Appel, Hans-Günter, Prof. Dr., 26419 Accum (DE)
(72) Erfinder: Laabs, Walter, Dr. med., D-26419 Schortens (DE); Appel, Hans-Günter, Prof. Dr., 26419 Accum (DE)
(74) Vertreter: Siewers, Gescha, Dr.

(56) Entgegenhaltungen:
- EP-A- 0 251 694
- WO-A-83/02900

## Beschreibung

Die Erfindung betrifft Absaugvorrichtungen für medizinische Zwecke, wie sie beispielsweise in der Chirurgie, Zahnmedizin und Intensivmedizin verwendet werden.

Bei Operationen müssen austretendes Blut, Gewebeflüssigkeit, Gewebefragmente, Knochensplitter und Knochenmehl, aber auch Eiter vom Operationsfeld entfernt werden, wobei hinzukommt, daß auch die üblichen Spülflüssigkeiten wie Kochsalz- oder Ringerlösung ständig erneuert und damit auch entfernt werden müssen. Früher verwendete man hierfür Mulltupfer, während heutzutage Absaugvorrichtungen eingesetzt werden. In der Zahnmedizin müssen z.B. Speichel, Blut und Bohrerreste abgesaugt werden. In der Intensivmedizin, Anästhesie und Endoskopie müssen Körperflüssigkeiten verschiedener Viskosität und Zusammensetzung abgesaugt werden (z.B. Schleim, Blut, Urin, Stuhl, Galle, Magensaft etc. auch Kontrastflüssigkeiten). Derartige Absaugvorrichtungen bestehen aus einer fest installierten oder mobilen Vakuumpumpe, die an einen Unterdrucksammelbehälter angeschlossen ist, der seinerseits über einen Überlaufschutz mit einer sogenannten Sekretflasche verbunden ist, die wiederum ihrerseits über den Absaugschlauch mit der Absaugspitze in Verbindung steht. Es gibt kleinlumige und großlumige Sauger, wobei man als großlumige Sauger solche bezeichnet, bei denen der Innendurchmesser größer als 4,9 mm ist. Diese Sauger können aus Kunststoff, aber auch aus Metall oder anderen Werkstoffen bestehen.

Wenn beim Absaugen Gewebe die Öffnung der Saugspitze des Absaugers vollständig bedeckt, bildet sich ein Unterdruck, der in der Größenordnung bis an den Pumpenunterdruck von 0,1 bar heranreichen kann. Dadurch wird Gewebe durch den Luftdruck in die Saugöffnung hineingedrückt, so daß Gewebeschädigungen entstehen können. Außerdem kann Gewebe am Innenrand der Saugöffnung abgeschert werden, was ebenfalls zu Verletzungen führen kann.

Um das Festsaugen der Saugspitze am Gewebe zu vermeiden, hat man zwischenzeitlich sogenannte atraumatische Sauger entwickelt, bei denen oberhalb der Saugspitze Seitenöffnungen vorhanden sind, durch die zusätzliche Luft angesaugt werden kann. Dies führt zwar einerseits zu einer Erniedrigung der Saugleistung, auf der anderen Seite wird aber der kritische Unterdruck in der Saugspitze und somit eine Verletzung bzw. ein Einsaugen von Gewebe vermieden oder reduziert. Um die Erniedrigung der Saugleistung möglichst klein zu halten, hat man auch bereits Sauger mit einer sogenannten Vakuumkontrolle entwickelt, die über eine seitliche Öffnung verfügen, die beim Absaugen mit einem Finger wie bei einer Flöte zugehalten werden muß und bei der durch Freigeben der Öffnung der Unterdruck am Gewebe abgebaut werden kann. Die Vakuumkontrolle muß allerdings mit der Hand erfolgen, wodurch derartige Absauger in der Handhabung Aufmerksamkeit erfordern und daher bei der Operation störend wirken können.

Außerdem hat sich herausgestellt, daß die sogenannten atraumatischen Sauger, bei denen sich Luftströme aus den Seitenbohrungen mit dem Hauptluftstrom mischen, zu einer stark störenden Geräuschentwicklung Anlaß geben. Diese Sauger funktionieren ja nach dem Prinzip der Blockflöte und die Geräusche entstehen, solange nicht gesaugt wird, durch die Verwirbelung der Seitenluft im Hauptluftstrom. Die Lautstärke wird durch die Luftgeschwindigkeit im Schlauch und durch Resonanzschwingungen des Saugers bestimmt; die Frequenz dieser Geräusche ist abhängig von der Form und Lage der Seitenbohrungen. Die so entstehenden Pfeiftöne stören die Konzentration des Operationsteams und können bei länger dauernder Operationen ausgesprochen lästig fallen.

Die Entwicklung ging daher zu Saugspitzen mit Unterdruckventilen wie z.B. aus der Patentschrift WO 83/02900 bekannt. Nachteil derartiger Einrichtungen ist der komplizierte Aufbau, der es nur schwer zuläßt, das Gerät zu reinigen und zu sterilisieren. Deshalb werden solche Geräte üblicherweise als Einweg-Saugspitzen ausgeführt, was die Kosten in die Höhe treibt und das Müllvolumen erheblich vergrößtert. Ein weiterer Nachteil ist der nicht regulierbare Grenzunterdruck, bei dem das Unterdruckventil öffnet. Da sich die Viskosität und Zusammensetzung der abzusaugenden Flüssigkeiten stark ändern kann, wäre es wünschenswert, den Öffnungsdruck des Ventiles während einer Operation den jeweiligen Gegebenheiten anpassen zu können.

Es besteht daher noch ein Bedarf an Absaugvorrichtungen der oben dargestellten Art, die nicht traumatisierend wirken, keine unerwünschte Geräuchskulisse verursachen, einfach aufgebaut und so bei Bedarf leicht zu reinigen und zu sterilisieren sind.

Erfindungsgemäß werden nun Absaugvorrichtungen mit einem Sauger aus Kunststoff oder Metall vorgeschlagen, die eine oder mehrere Seitenöffnungen mit einem auf Unterdruck ansprechenden Ventil besitzt und dadurch gekennzeichnet ist, daß die Seitenöffnungen als Langlöcher oder als durchgehende Nut ausgebildet und mit einer dünnen Schlitzfolie abgedeckt sind.

Wenn die Absaugvorrichtung in üblicherweise an die Vakuumpumpe angeschlossen ist, aber nicht gesaugt wird, ist die Luftdruckdifferenz gering genug, um dieses Schlitzventil geschlossen zu halten und damit den Eintritt von Seitenluft und damit die Geräuschentwicklung zu verhindern. In dem Augenblick aber, in dem Gewebe die Saugeröffnung verschließt und ein kritischer Unterdruck sich in dem Sauger zu bilden beginnt, kann Seitenluft durch das Schlitzventil eintreten, so daß der Unterdruck auf ein Maß abgesenkt wird, bei dem keine Schädigung des Gewebes auftritt. Die Saugleistung wird dadurch jedoch nur wenig herabgesetzt.

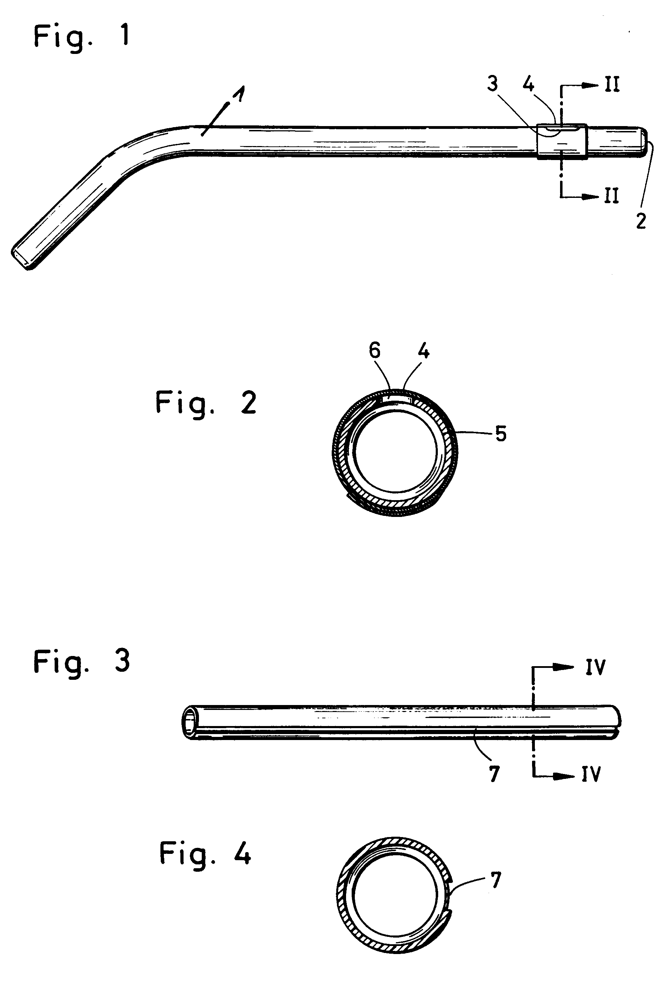

Wenn die Absaugvorrichtung aus Kunststoff besteht, kann in an sich bekannter Weise die Abdeckung des oder der Langlöcher bereits bei der Herstellung durch Spritzguß oder bei der Extrusion erfolgen. Extrudierte Sauger können auf ganzer Länge innen (Abb. 3) oder außen (Abb. 4) mit einer oder mehreren Nuten so versehen werden, daß eine membrandünne Wandschicht verbleibt. Diese Nut ersetzt das Langloch. Die Nut kann örtlich beliebig oft geschlitzt werden. Es ist aber auch möglich, daß wie im Fall der aus Metall bestehenden Absaugvorrichtungen über das Langloch eine dünne Folie geklebt oder ein festsitzender sehr dünner Schlauch mit einem Schlitz geschoben werden. Es ist sogar möglich, daß man über das Langloch eine Folie klebt oder einen Schlauch aufschiebt, der noch über keinen Schlitz verfügt, aber leicht einschneidbar ist, so daß während der Operation der Operateur den Ventilschlitz durch einfaches Trennen der Folie oder des Schlauches selbst herstellen kann, und zwar kann dann der gewünschte Unterdruck durch die veränderbare Länge des Ventilschlitzes reguliert werden.

Die Verwendung von Metallinstrumenten hat gegenüber Einwegartikel aus Kunststoff den Vorzug, daß sich diese Sauger durch ihre einfache Bauweise sehr gut reinigen und sterilisieren lassen, so daß nicht nur Kosten eingespart, sondern auch das anfallende Müllvolumen verkleinert werden können.

In der Regel sollten die Langlöcher oder Seitenöffnungen mit ihren Ventilen sich in einem großen Abstand von der Saugerspitze befinden, denn dadurch kann erreicht werden, daß beim Eintauchen die Ventile funktionsfähig bleiben. Bei tiefen Operationsfeldern, z.B. der Bauchhöhle ist es dagegen vorteilhaft mehrere Ventilöffnungen auf die ganze Saugerlänge anzuordnen. Werden Saugeröffnungen oder einzelne Ventilöffnungen z.B. durch Weichgewebe verstopft, können andere Ventilöffnungen in der Flüssigkeit die Saugfunktion aufrechterhalten.

Die Erfindung wird im folgenden anhand der Zeichnungen näher erläutert:
Abb. 1 zeigt einen erfindungsgemäßen Sauger in Seitenansicht.
Abb. 2 zeigt dieselben Sauger im Querschnitt.
Abb. 3 zeigt einen Sauger mit extrudierter Nut.
Abb. 4 zeigt einen Sauger nach Abb. 3 im Querschnitt.

Der Sauger (1) verfügt über eine Öffnung (2) und über ein oder mehrere Langlöcher (3), die ihrerseits mit einer bereits geschlitzten oder schlitzbaren Folie (4) abgedeckt sind. Aus der Querschnittszeichnung ergibt sich, daß die Wandung (5) der Sauger (1) im Bereich des Langloches (3) die Abdeckung durch die Folie (4) mit dem Schlitz (6) aufweist, wobei die zur Abdeckung benutzte Folie beispielsweise bei der Herstellung gleich bei Kunststoffteilen angespitzt oder mitextrudiert oder nachträglich als Schlauch über das Langloch übergezogen oder als Klebefolie übergeklebt werden kann.

Der Sauger (1) kann bei Kunststoff auch mit einer Nut (7) mit wesentlich geringerer Wandfläche versehen sein, wie dies im Detail in Abb. 4 dargestellt ist. Diese Nut kann in beliebiger Länge eingeschlitzt werden.

## Patentansprüche

1. Absaugvorrichtung für medizinische Zwecke, mit einem Sauger (1) aus Kunststoff oder Metall mit einer oder mehreren Seitenöffnungen (3) und mit auf Unterdruck ansprechenden Ventil (4), dadurch gekennzeichnet, daß die Seitenöffnungen (3) als Langlöcher (3) oder als durchgehende Nut (7) ausgebildet und mit einer dünnen Schlitzfolie (4) abgedeckt sind.

2. Absaugvorrichtung nach Anspruch 1, dadurch gekennzeichnet, daß die Langlöcher (3) in geringem Abstand von der Öffnung (2) des Saugers angeordnet sind.

3. Absaugvorrichtung nach Anspruch 1, dadurch gekennzeichnet, daß die Langlöcher (3) in großem Abstand von der Öffnung (2) des Saugers angeordnet sind.

4. Absaugvorrichtung nach Anspruch 1, dadurch gekennzeichnet, daß der aus Kunststoff bestehende Sauger (1) eine durchgehende Nut (7) mit membrandünner schlitzbarer Wandstärke aufweist.

## Claims

1. Suction device for medical purposes with a sucker (1) of plastic material or metal with one or several side-openings (3) and with a valve (4) reacting to subpressure characterized in that the side-openings (3) are long-holes or a continous nute (7) and are covered by a thin slottable film (4).

2. Suction device according to claim 1 characterized in that the long-holes are arranged at a short distance from the front opening (2) of the sucker.

3. Suction device according to claim 1 characterized in that the long-holes are arranged at a long distance from the front opening (2) of the sucker.

4. Suction device according to claim 1 characterized in that the sucker (1) consisting of plastic material has a continous nute (7) with membranous slottable wall thickness.

## Revendications

1. Dispositif de suction à usage médical avec un extracteur (1) en matière plastique ou en métal avec un ou plusieurs orifices latéraux (3) et avec une soupape (4) réagissant à vide partiel, caracterisé en ce que les orifices latéraux (3) sont des trous longitudinaux ou une rainure (7) continue et sont couverts par une feuille mince entaillable (4).

2. Dispositif de suction selon revendication 1 caracterisé en ce que les trous longitudinaux (3) sont arrangés en petite distance de l'ouverture (2) de l'extracteur.

3. Dispositif de suction selon revendication 1 caracterisé en ce que les trous longitudinaux (3) sont arrangés en grande distance de l'ouverture (2) de l'extracteur.

4. Dispositif de suction selon revendication 1 caracterisé en ce que l'extracteur (1) se composant de matière plastique a une rainure continue (7) avec une paroi entaillable d'épaisseur membraneuse.
